# FASCICULE DE BREVET EUROPEEN

(11) **EP 2 486 025 B1**
(45) Date de publication et mention de la délivrance du brevet: **10.08.2016**
(21) Numéro de dépôt: 10770752.3
(22) Date de dépôt: 08.10.2010
(51) Int. Cl.: C07D 285/125, C08J 3/24, C08K 5/00, C08K 5/46

(54) **THIADIAZOLE UTILISABLE COMME ACCÉLÉRATEUR DE VULCANISATION ET SON PROCÉDÉ D'OBTENTION.**
THIADIAZOL ZUR VERWENDUNG ALS VULKANISIERUNGSBESCHLEUNIGER UND VERFAHREN ZU DESSEN ERHALT
THIADIAZOLE WHICH CAN BE USED AS A VULCANIZATION ACCELERATOR AND METHOD FOR OBTAINING SAME

(30) Priorité: 08.10.2009 FR 0957038
(43) Date de publication de la demande: 15.08.2012
(73) Titulaire: Société de Technologie MICHELIN, 63000 Clermont-Ferrand (FR); MICHELIN Recherche et Technique S.A., 1763 Granges-Paccot (CH)
(72) Inventeur: SEEBOTH, Nicolas, F-63000 Clermont-Ferrand (FR); IVANOV, Sergey, Orekhovo-Zouevo 142611 (RU)
(74) Mandataire: Sidhu, Alban
(86) Numéro de dépôt international: PCT/EP2010/065070
(87) Numéro de publication internationale: WO 2011/042523

(56) Documents cités:
- DE-A1- 2 151 895
- FR-A1- 2 320 297
- US-A- 2 899 439
- US-A- 2 983 716

## Description

La présente invention se rapporte à un thiadiazole particulier, à son procédé d'obtention et à son utilisation en tant qu'accélérateur de vulcanisation.

La vulcanisation des élastomères diéniques par le soufre est largement utilisée dans l'industrie du caoutchouc, en particulier celle du pneumatique. Pour vulcaniser les élastomères diéniques, on utilise un système de vulcanisation relativement complexe comportant, en plus du soufre, un accélérateur primaire de vulcanisation, tels que les sulfénamides à noyau benzothiazole (DE2151895 A1), ainsi que divers accélérateurs secondaires ou activateurs de vulcanisation, tout particulièrement des dérivés du zinc tels que l'oxyde de zinc (ZnO) seul ou utilisé avec des acides gras.

Ces accélérateurs de vulcanisation doivent induire une réticulation suffisante tout en conservant un compromis acceptable entre les différentes propriétés rhéométriques.

La demanderesse a découvert un nouveau composé thiadiazole qui peut être utilisé comme accélérateur de vulcanisation de compositions caoutchouc. En particulier ce nouveau composé permet dans une utilisation pour des compositions de caoutchouc pour pneumatiques, d'obtenir un compromis de propriétés rhéométriques similaire à celui obtenu avec des accélérateurs de vulcanisation classiquement utilisés.

L'invention a donc notamment pour objet un thiadiazole de formule (I) : 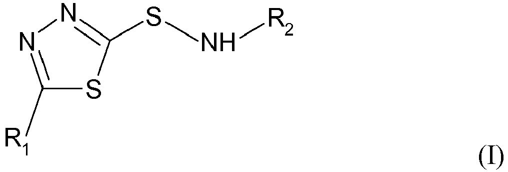 où
R₁ représente H ou un groupe hydrocarboné en C₁-C₂₅ choisi parmi les groupes alkyles linéaires, ramifiés ou cycliques, et les groupes aryles, éventuellement interrompus par un ou plusieurs hétéroatomes,
R₂ représente :
   - un groupe alkyle linéaire en C₂-C₂₅ ou ramifié en C₃ ou C₅-C₂₅ éventuellement interrompu par un ou plusieurs hétéroatomes, éventuellement substitué par un ou plusieurs groupes alkyles cycliques en C₃-C₁₀ ou aryles en C₆-C₁₂, ou
   - un groupe alkyle cyclique en C₃-C₁₀, éventuellement interrompu par un ou plusieurs hétéroatomes, éventuellement substitué par un ou plusieurs groupes alkyles linéaires, ramifiés ou cycliques en C₁-C₂₅ ou aryles en C₆-C₁₂, éventuellement interrompus par un ou plusieurs hétéroatomes.

L'invention a encore pour objet un procédé d'obtention d'un thiadiazole tel que défini précédemment, comprenant les étapes suivantes :
- on part du composé (A) de formule suivante : 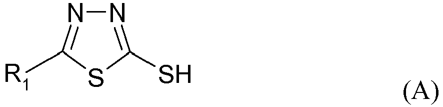 où R₁ est tel que défini précédemment,
- on fait réagir le composé (A) avec un composé de formule R₂NH₂, où R₂ est tel que défini précédemment, en présence d'une base organique ou minérale, puis
- on ajoute au milieu réactionnel une composition oxydante comprenant au moins un agent oxydant, pour obtenir le thiadiazole de formule (I).

L'invention a enfin pour objet l'utilisation, à titre d'accélérateur de vulcanisation, d'un thiadiazole tel que défini précédemment.

L'invention ainsi que ses avantages seront aisément compris à la lumière de la description et des exemples de réalisation qui suivent.

### I. Mesures et tests utilisés

Les compositions de caoutchouc, dans lesquelles sont testés les accélérateurs de vulcanisation thiadiazoles, sont caractérisées, avant et après cuisson, comme indiqué ci-après.

### Rhéométrie

Les mesures sont effectuées à 150°C avec un rhéomètre à chambre oscillante, selon la norme DIN 53529 - partie 3 (juin 1983). L'évolution du couple rhéométrique, ΔCouple, en fonction du temps décrit l'évolution de la rigidification de la composition par suite de la réaction de vulcanisation. Les mesures sont traitées selon la norme DIN 53529 - partie 2 (mars 1983) : T₀ est le délai d'induction, c'est-à-dire le temps nécessaire au début de la réaction de vulcanisation ; T_{α} (par exemple T₉₉) est le temps nécessaire pour atteindre une conversion de α%, c'est-à-dire α% (par exemple 99%) de l'écart entre les couples minimum et maximum. On mesure également la constante de vitesse de conversion notée K (exprimée en min-1), d'ordre 1, calculée entre 30% et 80% de conversion, qui permet d'apprécier la cinétique de vulcanisation.

### II. Conditions de réalisation de l'invention

### II-1. Thiadiazole de l'invention

Comme expliqué précédemment, le premier objet de l'invention est un thiadiazole de formule (I) suivante : 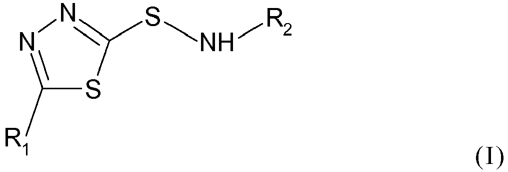 où
R₁ représente H ou un groupe hydrocarboné en C₁-C₂₅ choisi parmi les groupes alkyles linéaires, ramifiés ou cycliques, et les groupes aryles, éventuellement interrompus par un ou plusieurs hétéroatomes,
R₂ représente :
   - un groupe alkyle linéaire en C2-C25 ou ramifié en C3 ou C5-C25 éventuellement interrompu par un ou plusieurs hétéroatomes, éventuellement substitué par un ou plusieurs groupes alkyles cycliques en C₃-C₁₀ ou aryles en C₆-C₁₂, ou
   - un groupe alkyle cyclique en C₃-C₁₀, éventuellement interrompu par un ou plusieurs hétéroatomes, éventuellement substitué par un ou plusieurs groupes alkyles linéaires, ramifiés ou cycliques en C₁-C₂₅ ou aryles en C₆-C₁₂, éventuellement interrompus par un ou plusieurs hétéroatomes.

Par groupe alkyle cyclique, on entend un groupe alkyle constitué d'un ou plusieurs cycles.

Le ou les hétéroatomes peuvent être un atome d'azote, de soufre ou d'oxygène.

De préférence, le composé thiadiazole de formule (I) est tel que :
R₁ représente H ou un groupe hydrocarboné en C₁-C₂₅ choisi parmi les groupes alkyles linéaires, ramifiés ou cycliques, et les groupes aryles, éventuellement interrompus par un ou plusieurs hétéroatomes,
R₂ représente :
   - un groupe alkyle linéaire en C₂-C₂₅ ou ramifié en C₃ ou C₅-C₂₅, éventuellement interrompu par un ou plusieurs hétéroatomes, éventuellement substitué par un ou plusieurs groupes alkyles cycliques en C₃-C₁₀ ou aryles en C₆-C₁₂, ou
   - un groupe alkyle cyclique en C₃-C₁₀, éventuellement interrompu par un ou plusieurs hétéroatomes, éventuellement substitué par un ou plusieurs groupes alkyles linéaires, ramifiés ou cycliques en C₁-C₂₅ ou aryles en C₆-C₁₂, éventuellement interrompus par un ou plusieurs hétéroatomes.

Selon un premier mode de réalisation, R₁ représente un groupe méthyle.

Selon un deuxième mode de réalisation, R₁ représente H.

R₂ représente avantageusement un groupe alkyle cyclique en C₃-C₁₀.

En particulier, R₂ peut représenter un groupe cyclohexyle.

Ainsi, un composé de formule (I) préféré est celui dans lequel R₁ représente H et R₂ représente un cyclohexyle. Dans ce cas, le composé thiadiazole de formule (I) est la N-cyclohexyl-S-(1,3,4-thiadiazol-2-yl)thiohydroxylamine.

Un autre composé de formule (I) préféré est celui dans lequel R₁ représente un méthyle et R₂ représente un cyclohexyle. Dans ce cas, le composé thiadiazole de formule (I) est la N-cyclohexyl-S-(5-méthyl-1,3,4-thiadiazol-2-yl)thiohydroxylamine.

### II-2. Procédé de synthèse

Selon l'invention, le procédé d'obtention d'un thiadiazole de formule (I) telle que défini précédemment comprend les étapes suivantes :
- on part du composé (A) de formule suivante : 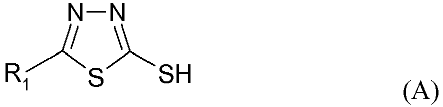 où R₁ est tel que défini précédemment,
- on fait réagir le composé (A) avec un composé de formule R₂NH₂, où R₂ est tel que défini précédemment, en présence d'une base organique ou minérale, puis
- on ajoute au milieu réactionnel une composition oxydante comprenant au moins un agent oxydant, pour obtenir le thiadiazole de formule (I).

Selon un premier mode de réalisation, R₁ représente l'hydrogène.

Selon un second mode de réalisation, R₁ représente un groupe méthyle.

De préférence, R₂ représente un groupe cyclohexyle.

Lorsque l'on fait réagir le composé (A) en présence d'une base, celle-ci peut être par exemple une solution aqueuse d'hydroxyde de sodium.

Comme expliqué précédemment, le procédé selon l'invention comprend une étape d'ajout d'une composition oxydante. L'agent oxydant peut être choisi parmi les oxydants classiques tels que le brome, le chlore, l'iode ou encore les acides hypobromique, hypochlorique ou hypoiodique ou bien encore les sels alcalins des acides précédents. Généralement, une solution aqueuse d'hypochlorite de sodium est préférée.

### II-3. Utilisation à titre d'accélérateur de vulcanisation

Comme indiqué précédemment, le composé thiadiazole de l'invention trouve une application industrielle avantageuse comme accélérateur de vulcanisation. Il peut donc être utilisé dans une composition de caoutchouc pour la fabrication de pneumatiques, à base d'un ou plusieurs élastomères diéniques, d'une ou plusieurs charges renforçantes et d'un système de vulcanisation.

Pour une telle utilisation, le ou les élastomères diéniques sont choisis préférentiellement dans le groupe des élastomères diéniques fortement insaturés constitué par les polybutadiènes (en abrégé "BR"), les polyisoprènes (IR) de synthèse, le caoutchouc naturel (NR), les copolymères de butadiène, les copolymères d'isoprène et les mélanges de ces élastomères. De tels copolymères sont plus préférentiellement choisis dans le groupe constitué par les copolymères de butadiène-styrène (SBR), les copolymères d'isoprène-butadiène (BIR), les copolymères d'isoprène-styrène (SIR) et les copolymères d'isoprène-butadiène-styrène (SBIR).

Par ailleurs, on peut utiliser tout type de charge renforçante connue pour ses capacités à renforcer une composition de caoutchouc utilisable pour la fabrication de pneumatiques, par exemple une charge organique tel que du noir de carbone, une charge inorganique renforçante telle que de la silice, ou encore un coupage de ces deux types de charge, notamment un coupage de noir de carbone et de silice.

Par "charge inorganique renforçante", doit être entendu dans la présente demande, par définition, toute charge inorganique ou minérale (quelles que soient sa couleur et son origine (naturelle ou de synthèse), encore appelée charge "blanche", charge "claire" voire "charge non noire" ("non-black filler") par opposition au noir de carbone, capable de renforcer à elle seule, sans autre moyen qu'un agent de couplage intermédiaire, une composition de caoutchouc destinée à la fabrication de pneumatiques, en d'autres termes apte à remplacer, dans sa fonction de renforcement, un noir de carbone conventionnel de grade pneumatique ; une telle charge se caractérise généralement, de manière connue, par la présence de groupes hydroxyle (-OH) à sa surface.

Comme charges inorganiques renforçantes conviennent notamment des charges minérales du type siliceuse, en particulier de la silice (SiO₂).

Le système de vulcanisation proprement dit est à base de soufre (ou d'un agent donneur de soufre) et d'un accélérateur primaire de vulcanisation. A ce système de vulcanisation de base viennent s'ajouter divers accélérateurs secondaires ou activateurs de vulcanisation connus tels qu'oxyde de zinc, acide stéarique ou composés équivalents, dérivés guanidiques (en particulier diphénylguanidine).

L'accélérateur primaire de vulcanisation doit permettre une réticulation des compositions de caoutchouc dans des temps industriellement acceptables, tout en préservant un délai minimum de sécurité (« temps de grillage ») au cours duquel les compositions peuvent être mises en forme sans risque de vulcanisation prématurée (« grillage »).

Le composé thiadiazole selon l'invention peut donc être utilisé à titre d'accélérateur de vulcanisation. Il remplace en tout ou partie les composés sulfénamides habituels.

### III. Exemples de réalisation de l'invention

Dans les exemples qui suivent, la synthèse de deux composés thiadiazole particuliers selon l'invention est présentée, puis l'invention est mise en oeuvre avec la N-cyclohexyl-S-(1,3,4-thiadiazol-2-yl)thiohydroxylamine (composé B).

### III-1. Synthèse du composé N-cyclohexyl-S-(1,3,4-thiadiazol-2-yl)thiohydroxylamine (composé B)

Le composé B a pour formule :

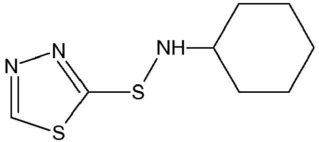

La préparation de ce composé est effectuée à partir du 1,3,4-thiadiazole-2-thiol et de la cyclohexylamine, selon le schéma de synthèse suivant :

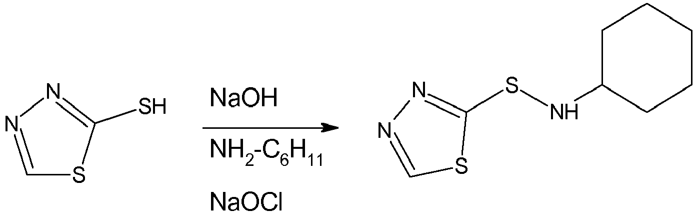

Le 1,3,4-thiadiazole-2-thiol est commercial (numéro CAS [18686-82-3]. Il peut être obtenu à partir de disulfure de carbone et d'hydrate d'hydrazine selon des procédures décrites dans les documents suivants :
1. CH 563 380 (1971)
2. FR 71 47384 (1972)

Le schéma réactionnel de la préparation du 1,3,4-thiadiazole-2-thiol est le suivant :

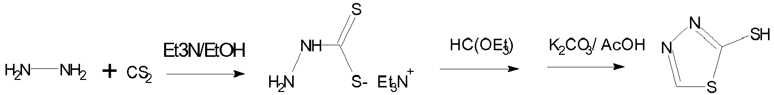

A une solution de 1,3,4-thiadiazole-2-thiol (18,44 g, 0,16 mol) et d'hydroxyde de sodium (14,04 g, 0,35 mol) dans l'eau (900 mL) est ajouté la cyclohexylamine (77,35 g, 0,78 mol). Le mélange est refroidi jusqu'à 0 - 5°C puis est ajoutée goutte à goutte la solution aqueuse de NaOCl (4 % de chlore actif) (343 mL) pendant 15 minutes. La température du milieu réactionnel est maintenue entre 0 et + 5°C. Le milieu réactionnel est ensuite agité pendant une à une heure et demi à une température comprise entre 0 et 5°C.

De l'éther de pétrole (100 mL) est ajouté et le milieu réactionnel est ensuite agité pendant 15 à 30 minutes à une température comprise entre 0 et -4°C. Le précipité est filtré et lavé par de l'eau (200 mL) et de l'éther de pétrole (50 mL) et puis séché pendant 2 à 3 heures sous pression réduite et 12 heures à température ambiante.

Un solide blanc (11,0 g, 0,05 mol) de point de fusion 81-83 °C est obtenu.

La pureté molaire est supérieure à 96 % (RMN ¹H).

La caractérisation complète du produit est réalisée par RMN. Les déplacements chimiques obtenus par RMN 1H dans l'acétone-d6 sont donnés dans le tableau ci-dessous. La calibration est réalisée sur l'acétone (1,98 ppm en ¹H)

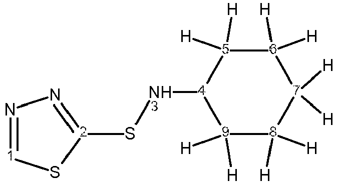

| | 1 | 2 | 3 | 4 | 5 | 6 | 7 | 8 | 9 |
|---|---|---|---|---|---|---|---|---|---|
| δ ¹H (ppm) | 9,21 | - | 4,72 | 2,81 | 1,98 | 1,67 | 1,52 | 1,67 | 1,98 |
| | | | | | 1,19 | 1,25 | 1,14 | 1,25 | 1,19 |

### III-2. Synthèse du composé N-cyclohexyl-S-(5-méthyl-1,3,4-thiadiazol-2-yl)thiohydroxylamine

Le N-cyclohexyl-S-(5-méthyl-1,3,4-thiadiazol-2-yl)thiohydroxylamine a pour formule :

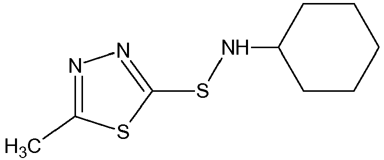

La préparation de ce composé est effectuée à partir du 5-méthyl-1,3,4-thiadiazole-2-thiol et de la cyclohexylamine, selon le schéma de synthèse suivant :

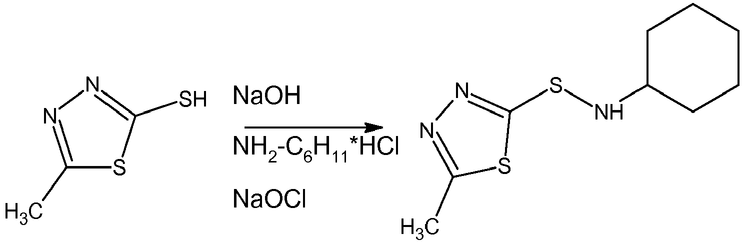

Le 5-méthyl-1,3,4-thiadiazole-2-thiol est commercial (numéro CAS [29490-19-5]. Il peut être obtenu à partir de disulfure de carbone et d'acétyl hydrazine selon des procédures décrites dans les documents suivants :
1. CH 563 380 (1971)
2. FR 71 47384 (1972)

Le schéma réactionnel de la préparation du 5-méthyl-1,3,4-thiadiazole-2-thiol est le suivant :

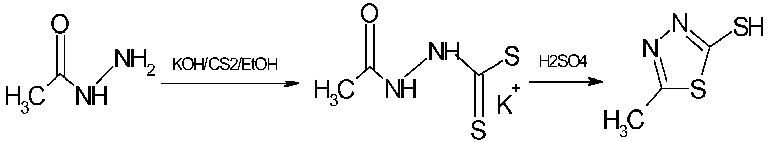

A une solution de 5-methyl-1,3,4-thiadiazole-2-thiol (37,50 g, 0,28 mol) et d'hydroxyde de sodium (85,20 g, 2,13 mol) dans l'eau (1 L) est ajouté le chlorhydrate de cyclohexylamine (192,30 g, 1,42 mol). Le mélange est refroidi jusqu'à une température comprise entre 0 et - 5°C puis est ajoutée goutte à goutte la solution aqueuse de NaOCl (4 % du chlore actif) (600 mL) pendant 15 à 30 minutes. La température du milieu réactionnel est maintenue entre 0 et + 5°C. Le milieu réactionnel est ensuite agité pendant 1h30 à 2 heures à une température comprise entre 0 et + 5°C.

De l'éther de pétrole (100 mL) est ajouté et le milieu réactionnel est ensuite agité pendant 1h30 à 2 heures à une température comprise entre 0 et -4°C. Le précipité est filtré et lavé par de l'eau (200 mL) et de l'éther de pétrole (50 mL) et enfin séché pendant 2 à 3 heures sous pression réduite et 12 heures à température ambiante.

Un solide blanc (23,27 g, 0,10 mol) de point de fusion 94-96 °C est obtenu.

La pureté molaire est supérieure à 97 % (RMN ¹H).

La caractérisation complète du produit est réalisée par RMN. Les déplacements chimiques obtenus par RMN 1H dans l'acétone-d6 sont donnés dans le tableau ci-dessous. La calibration est réalisée sur l'acétone (1,98 ppm en ¹H)

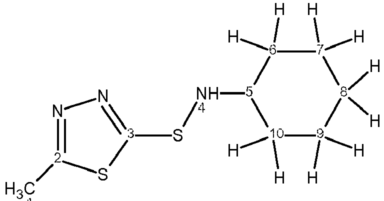

| | 1 | 2 | 3 | 4 | 5 | 6 | 7 | 8 | 9 | 10 |
|---|---|---|---|---|---|---|---|---|---|---|
| δ ¹H (ppm) | 2,57 | - | - | 5,40 | 2,68 | 1,84 | 1,60 | 1,46 | 1,60 | 1,84 |
| | | | | | | 1,11 | 1,16 | 1,06 | 1,16 | 1,11 |

### III-3. Utilisation comme accélérateur de vulcanisation-Préparation des compositions

On procède pour les essais qui suivent de la manière suivante: on introduit dans un mélangeur interne, rempli à 70% et dont la température initiale de cuve est d'environ 90°C, le ou les élastomères diéniques, la ou les charges renforçantes, l'agent de couplage éventuel puis, après une à deux minutes de malaxage, les divers autres ingrédients à l'exception du système de vulcanisation. On conduit alors un travail thermomécanique (phase non-productive) en une étape (durée totale du malaxage égale à environ 5 min), jusqu'à atteindre une température maximale de "tombée" d'environ 165°C. On récupère le mélange ainsi obtenu, on le refroidit puis on ajoute l'agent de recouvrement (lorsque ce dernier est présent) et le système de vulcanisation (soufre et composé thiadiazole (ou « CBS » pour l'exemple comparatif)) sur un mélangeur externe (homo-finisseur) à 70°C, en mélangeant le tout (phase productive) pendant environ 5 à 6 min.

Les compositions ainsi obtenues sont ensuite calandrées soit sous la forme de plaques (épaisseur de 2 à 3 mm) ou de feuilles fines de caoutchouc pour la mesure de leurs propriétés physiques ou mécaniques, soit sous la forme de profilés utilisables directement, après découpage et/ou assemblage aux dimensions souhaitées, par exemple comme produits semi-finis pour pneumatiques, en particulier comme bandes de roulement de pneumatiques.

### III-4. Essais de caractérisation - Résultats

L'objet de cet exemple est de comparer les propriétés rhéométriques d'une composition de caoutchouc utilisable pour la fabrication d'une bande de roulement de pneumatique, comprenant la N-cyclohexyl-S-(1,3,4-thiadiazol-2-yl)thiohydroxylamine (composé B) à titre d'accélérateur primaire de vulcanisation (composition 2) avec les propriétés d'une composition de caoutchouc comprenant la N-cyclohexyl-2-benzothiazyle sulfénamide (« CBS ») (composition 1).

Les formulations des compositions sont données dans le tableau 1. Les quantités sont exprimées en parties pour 100 parties en poids d'élastomère (pce).

**Tableau 1**

| | Composition 1 | Composition 2 |
|---|---|---|
| NR (1) | 100 | 100 |
| N220 (2) | 47,5 | 47,5 |
| Paraffine | 1 | 1 |
| TMQ (3) | 1 | 1 |
| 6-PPD (4) | 1,5 | 1,5 |
| Acide stéarique | 2,5 | 2,5 |
| ZnO | 2,7 | 2,7 |
| Soufre | 1,5 | 1,5 |
| Accélérateur de vulcanisation | 0,6 * | 0,51 ** |

| | | |
|---|---|---|
| * CBS (« Santocure CBS » de la société Flexsys) ** N-cyclohexyl-S-(1,3,4-thiadiazol-2-yl)thiohydroxylamine (1) caoutchouc naturel (2) noir de carbone (3) TMQ :2,2,4-trimethyl-1,2-dihydroquinoline commercialisé par la société Flexys (4) Agent anti-oxydant 6-para-phénylènediamine | | |

La composition de caoutchouc 2 comprenant la N-cyclohexyl-S-(1,3,4-thiadiazol-2-yl)thiohydroxylamine est identique à la composition 1, étant entendu que la CBS est remplacée par une quantité isomolaire de N-cyclohexyl-S-(1,3,4-thiadiazol-2-yl)thiohydroxylamine.

Les propriétés rhéométriques à 150°C sont données dans le tableau 2.

**Tableau 2**

| | Composition 1 (CBS) | Composition 2 (composé B) |
|---|---|---|
| Prop.rhéo. *(DIN)* | 150°C | |
| Δcouple(dN.m) | 7,0 | 6,3 |
| k(min⁻¹) | 0,323 | 0,208 |
| t₀(min) | 4,9 | 3,2 |
| t₉₉ (min) | 19,2 | 25,3 |

Les propriétés rhéométriques obtenues pour la composition contenant la N-cyclohexyl-S-(1,3,4-thiadiazol-2-yl)thiohydroxylamine sont équivalentes à celles obtenues pour la composition contenant la CBS. On constate donc que l'utilisation de la N-cyclohexyl-S-(1,3,4-thiadiazol-2-yl)thiohydroxylamine à titre d'accélérateur d'une composition de caoutchouc pour pneumatique permet d'obtenir un compromis sur les différentes propriétés rhéométriques similaire à celui obtenu avec des accélérateurs de vulcanisation classiquement utilisés pour cette application.

On note, par ailleurs que le composé B, ainsi que les composés de formule (I) en général, remplacent avantageusement vis-à-vis de l'impact environnemental, les sulfénamides à noyau mercaptobenzothiazole, en ne générant pas contrairement à ces derniers, de mercaptobenzothiazole en se décomposant au cours de la cuisson.

## Revendications

1. Thiadiazole de formule (I) : 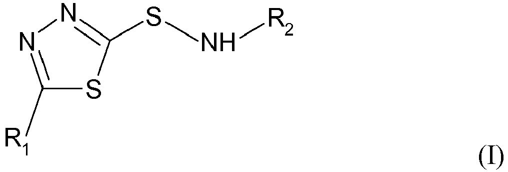 où
R₁ représente H ou un groupe hydrocarboné en C₁-C₂₅ choisi parmi les groupes alkyles linéaires, ramifiés ou cycliques, et les groupes aryles, éventuellement interrompus par un ou plusieurs hétéroatomes,
R₂ représente :
- un groupe alkyle linéaire en C₂-C₂₅ ou ramifié en C₃ ou C₅-C₂₅, éventuellement interrompu par un ou plusieurs hétéroatomes, éventuellement substitué par un ou plusieurs groupes alkyles cycliques en C₃-C₁₀ ou aryles en C₆-C₁₂, ou
- un groupe alkyle cyclique en C₃-C₁₀, éventuellement interrompu par un ou plusieurs hétéroatomes, éventuellement substitué par un ou plusieurs groupes alkyles linéaires, ramifiés ou cycliques en C₁-C₂₅ ou aryles en C₆-C₁₂, éventuellement interrompus par un ou plusieurs hétéroatomes.

2. Thiadiazole selon la revendication 1 **caractérisée en ce que** R₁ représente H.

3. Thiadiazole selon la revendication 2 **caractérisée en ce que** R₁ représente un groupe méthyle.

4. Thiadiazole selon l'une quelconque des revendications précédentes **caractérisée en ce que** R₂ représente un groupe cyclohexyle.

5. Procédé d'obtention d'un thiadiazole telle que défini dans l'une quelconque des revendications précédentes **caractérisé en ce qu'**il comprend les étapes suivantes :
- on part du composé (A) de formule suivante : 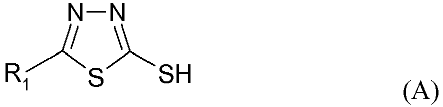 où R₁ est tel que défini à l'une quelconque des revendications 1 à 3,
- on fait réagir le composé (A) avec un composé de formule R₂NH₂, où R₂ est tel que défini à la revendication 1 ou 4, en présence d'une base, puis
- on ajoute au milieu réactionnel une composition oxydante comprenant au moins un agent oxydant, pour obtenir le thiadiazole de formule (I).

6. Procédé selon la revendication 5 **caractérisé en ce que** la base est l'hydroxyde de sodium.

7. Procédé selon l'une quelconque des revendications 5 ou 6 **caractérisé en ce que** l'agent oxydant est choisi parmi l'iode et l'hypochlorite de sodium.

8. Utilisation, à titre d'accélérateur de vulcanisation, d'un thiadiazole telle que défini à l'une quelconque des revendications 1 à 4.

## Patentansprüche

1. Thiadiazol der Formel (I): 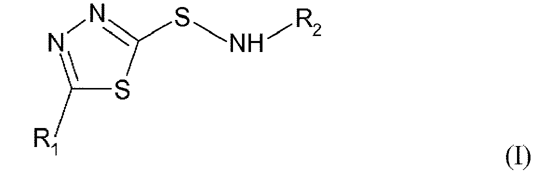 wobei
R₁ für H oder eine C₁-C₂₅-Kohlenwasserstoffgruppe, die aus linearen, verzweigten oder cyclischen Alkylgruppen und Arylgruppen, die gegebenenfalls durch ein oder mehrere Heteroatome unterbrochen sind, ausgewählt ist, steht,
R₂ für:
- eine lineare C₂-C₂₅- oder verzweigte C₃- oder C₅-C₂₅-Alkylgruppe, die gegebenenfalls durch ein oder mehrere Heteroatome unterbrochen ist und gegebenenfalls durch eine oder mehrere cyclische C₃-C₁₀-Alkyl- oder C₆-C₁₂-Arylgruppen substituiert ist, oder
- eine cyclische C₃-C₁₀-Alkylgruppe, die gegebenenfalls durch ein oder mehrere Heteroatome unterbrochen ist und gegebenenfalls durch eine oder mehrere lineare, verzweigte oder cyclische C₁-C₂₅-Alkyl- oder C₆-C₁₂-Arylgruppen, die gegebenenfalls durch ein oder mehrere Heteroatome unterbrochen sind, substituiert ist,
steht.

2. Thiadiazol nach Anspruch 1, **dadurch gekennzeichnet, dass** R₁ für H steht.

3. Thiadiazol nach Anspruch 2, **dadurch gekennzeichnet, dass** R₁ für eine Methylgruppe steht.

4. Thiadiazol nach einem der vorhergehenden Ansprüche, **dadurch gekennzeichnet, dass** R₂ für eine Cyclohexylgruppe steht.

5. Verfahren zum Erhalt eines Thiadiazols gemäß einem der vorhergehenden Ansprüche, **dadurch gekennzeichnet, dass** man:
- von der Verbindung (A) der folgenden Formel ausgeht: 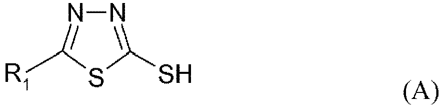 wobei R₁ wie in einem der Ansprüche 1 bis 3 definiert ist,
- die Verbindung (A) in Gegenwart einer Base mit einer Verbindung der Formel R₂NH₂, wobei R₂ wie in Anspruch 1 oder 4 definiert ist, umsetzt und dann
- eine oxidierend wirkende Zusammensetzung, die mindestens ein Oxidationsmittel umfasst, zum Reaktionsmedium gibt, wobei man das Thiadiazol der Formel (I) erhält.

6. Verfahren nach Anspruch 5, **dadurch gekennzeichnet, dass** es sich bei der Base um Natriumhydroxid handelt.

7. Verfahren nach einem der Ansprüche 5 oder 6, **dadurch gekennzeichnet, dass** das Oxidationsmittel aus Iod und Natriumhypochlorit ausgewählt wird.

8. Verwendung eines Thiadiazols gemäß einem der Ansprüche 1 bis 4 als Vulkanisationsbeschleuniger.

## Claims

1. Thiadiazole of formula (I): 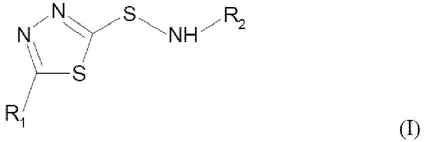 where
R₁ represents H or a C₁-C₂₅ hydrocarbon group chosen from linear, branched or cyclic alkyl groups and aryl groups which are optionally interrupted by one or more heteroatoms,
R₂ represents:
- a linear C₂-C₂₅ or branched C₃ or C₅-C₂₅ alkyl group which is optionally interrupted by one or more heteroatoms and which is optionally substituted by one or more cyclic C₃-C₁₀ alkyl or C₆-C₁₂ aryl groups, or
- a cyclic C₃-C₁₀ alkyl group which is optionally interrupted by one or more heteroatoms and which is optionally substituted by one or more linear, branched or cyclic C₁-C₂₅ alkyl or C₆-C₁₂ aryl groups which are optionally interrupted by one or more heteroatoms.

2. Thiadiazole according to Claim 1, **characterized in that** R₁ represents H.

3. Thiadiazole according to Claim 2, **characterized in that** R₁ represents a methyl group.

4. Thiadiazole according to any one of the preceding claims, **characterized in that** R₂ represents a cyclohexyl group.

5. Process for the preparation of a thiadiazole as defined in any one of the preceding claims, **characterized in that** it comprises the following stages:
- the starting compound is compound (A) of following formula: 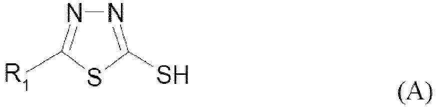 where R₁ is as defined in any one of Claims 1 to 3,
- compound (A) is reacted with a compound of formula R₂NH₂, where R₂ is as defined in Claim 1 or 4, in the presence of a base, then
- an oxidizing composition comprising at least one oxidizing agent is added to the reaction medium, in order to obtain the thiadiazole of formula (I).

6. Process according to Claim 5, **characterized in that** the base is sodium hydroxide.

7. Process according to either one of Claims 5 and 6, **characterized in that** the oxidizing agent is chosen from iodine and sodium hypochlorite.

8. Use, as vulcanization accelerator, of a thiadiazole as defined in any one of Claims 1 to 4.
